# EUROPEAN PATENT SPECIFICATION

(11) **EP 4 545 129 B1**
(45) Date of publication and mention of the grant of the patent: **22.07.2026**
(21) Application number: 24206423.6
(22) Date of filing: 14.10.2024
(51) Int. Cl.: A61M 25/02

(54) **CATHETER SECUREMENT DEVICE WITH ENERGY DISSIPATING BODY**
KATHETERBEFESTIGUNGSVORRICHTUNG MIT ENERGIEABLEITENDEM KÖRPER
DISPOSITIF DE FIXATION DE CATHÉTER AVEC CORPS DE DISSIPATION D'ÉNERGIE

(30) Priority: 24.10.2023 US 202318492952
(43) Date of publication of application: 30.04.2025
(73) Proprietor: B. BRAUN MEDICAL INC., Bethlehem Pennsylvania 18018-3524 (US)
(72) Inventor: Klein, Jason, Irvine (CA); Tomic-Edgar, Kerry, Irvine (CA); Beran, Anthony, Irvine (CA)
(74) Representative: Patentanwälte Ruff, Wilhelm, Beier, Dauster & Partner mbB

(56) References cited:
- CA-A1- 2 974 174
- US-A1- 2020 297 976
- US-A1- 2023 114 722
- US-B2- 10 463 836

## Description

### FIELD

The present disclosure relates generally to catheter devices, and more particularly to a device for securing the position of a smaller catheter, such as an epidural pain block catheter, and preventing migration of the catheter while it is inserted in a patient.

### BACKGROUND

Catheter tubes and lines are often inserted into patients and left in place for a significant amount of time. Depending on the medical condition, the patient may be free to move while the catheter is in place. In such cases, the catheter must remain in the proper position while the patient moves. In addition, the catheter insertion site must be protected from infection at all times.

Securement devices have been developed in an attempt to fix the position of catheters and prevent undesired movement. Many such devices are designed for larger catheters that have hubs or tubing connectors near the insertion site. These catheters are relatively easy to secure, because the hubs and tubing connectors are rigid and have surfaces that can be clamped or otherwise engaged by securement devices.

Other types of catheters do not feature rigid hubs or connectors near the insertion site, and therefore are more difficult to secure in place. For example, epidural pain block catheters typically do not have rigid hubs or connectors near the insertion site. In addition, they typically feature very thin tubes with diameters of approximately 2 mm or less. This makes it difficult to secure the catheter tube for a host of reasons. Securement devices that are designed to hold catheter hubs are too large and bulky to secure smaller tube diameters. Many securement devices also have rigid parts with edges that can pinch smaller diameter tubes. Moreover, many securement devices have flat rigid surfaces that allow smaller tubes to slide easily. This can promote gradual migration of the catheter over time.

For the foregoing reasons, conventional catheter securement devices are not adequate in all instances where catheter tubes are used.

Exemplary prior art catheter securement devices are disclosed in US10 463 836, US2020/297976, CA2974174 and US2023/114722.

### SUMMARY

The present disclosure describes a catheter securement device or "CSD" that can be used with many types of catheters, particularly those that lack hubs or connectors near the insertion site, have smaller diameters, and/or are otherwise difficult to secure with existing securement devices.

The invention is defined by claim 1; preferable embodiments are defined in the dependent claims.

In one aspect of the disclosure, a catheter securement device includes a base defining a slot and at least one inertial damper attached to the base and extending above the slot. The slot and the at least one inertial damper are relatively positioned so that when the slot is aligned over a catheter insertion site. A catheter tube exiting the catheter insertion site is receivable through the slot and coilable around at least a portion of the at least one inertial damper.

In another aspect of the disclosure, the slot and the at least one inertial damper are relatively positioned so that the catheter tube is coilable around the at least one inertial damper to fully surround the at least one inertial damper.

In another aspect of the disclosure, the at least one inertial damper includes a peripheral sidewall configured to frictionally engage the catheter tube.

In another aspect of the disclosure, the sidewall is compressible under stored energy.

In another aspect of the disclosure, the slot and the peripheral sidewall are relatively positioned so that when the slot is aligned over the catheter insertion site, the catheter tube exiting the catheter insertion site is receivable through the slot and coilable around at least a portion of the peripheral sidewall.

In another aspect of the disclosure, the peripheral sidewall includes a plurality of sidewall sections.

In another aspect of the disclosure, the slot and the peripheral sidewall are relatively positioned so that when the slot is aligned over the catheter insertion site, the catheter tube exiting the catheter insertion site is receivable through the slot and coilable around some or all of the plurality of sidewall sections.

In another aspect of the disclosure, the catheter securement device includes at least two coil retaining members configured to keep the catheter tube coiled around the at least one inertial damper.

In another aspect of the disclosure, each of the at least two coil retaining members is configured to limit migration of the catheter tube away from the sidewall after the catheter tube is coiled around the at least one inertial damper.

In another aspect of the disclosure, the at least two retaining members include at least two posts.

In another aspect of the disclosure, the catheter securement device includes a cover, and the at least one inertial damper is contained between the base and the cover.

In another aspect of the disclosure, the at least one inertial damper separates the base from the cover by a gap that surrounds the at least one inertial damper, the gap adapted to receive the catheter tube as the catheter tube is coiled around the at least one inertial damper.

In another aspect of the disclosure, the cover is tiltable relative to the base to increase the size of the gap on one side of the at least one inertial damper.

In another aspect of the disclosure, each post is configured to limit tilting of the cover relative to the base.

In another aspect of the disclosure, the at least one inertial damper includes a first lateral inertial damper, a second lateral inertial damper, and at least one medial inertial damper between the first lateral inertial damper and the second lateral inertial damper.

In another aspect of the disclosure, the at least two coil retaining members include the first lateral inertial damper and the second lateral inertial damper.

In another aspect of the disclosure, the first lateral inertial damper and the at least one medial inertial damper are separated by a first channel.

In another aspect of the disclosure, the second lateral inertial damper and the at least one medial inertial damper are separated by a second channel.

In another aspect of the disclosure, the first channel and the second channel are each configured such that the catheter tube is coilable around the at least one medial inertial damper through the first channel and the second channel.

In another aspect of the disclosure, the at least one medial inertial damper includes a first medial inertial damper and a second medial inertial damper.

In another aspect of the disclosure, the first medial inertial damper and the second medial inertial damper are separated by a third channel

In another aspect of the disclosure, the first channel, the second channel and the third channel are each configured such that the catheter tube is coilable around the first medial inertial damper and the second medial inertial damper through the first channel, the second channel and the third channel.

In another aspect of the disclosure, the first channel, the second channel and the third channel are parallel.

In another aspect of the disclosure, the slot intersects the third channel.

In another aspect of the disclosure, the catheter securement device includes a clear dressing configured to cover the base, the at least one inertial damper and the slot.

### BRIEF DESCRIPTION OF THE DRAWINGS

The drawing figures depict one or more implementations by way of example only, not by way of limitations. In the figures, like reference numerals can refer to the same or similar elements.
Fig. 1 is a schematic top view of a CSD according to the present disclosure, where the CSD is shown securing a catheter tube to a patient.
Fig. 2 is a front view of the CSD of Fig. 1.
Fig. 3 is an exploded front view of the CSD of Fig. 1.
Fig. 4 is a top view of a first component of the CSD of Fig. 1.
Fig. 5 is a top view of a second component of the CSD of Fig. 1.
Fig. 6 is a bottom view of a third component of the CSD of Fig. 1.
Fig. 7 is a schematic top view of the CSD of Fig. 1, showing one technique for securing a catheter tube to the CSD.
Fig. 8 is a block diagram describing a method for using a CSD according to the present disclosure.
Fig. 9 is a schematic top view of another CSD according to the present disclosure, where the CSD is shown in a closed state and securing a catheter tube to a patient.
Fig. 10 is a truncated perspective view of components of the CSD of Fig. 9.
Fig. 11 is an exploded front view of the CSD of Fig. 9.
Fig. 12 is a truncated top view of components of the CSD of Fig. 9.
Fig. 13 is a schematic top view of the CSD of Fig. 9, where the CSD is shown in an open state and securing a catheter tube to a patient.
Fig. 14 is a truncated top view of components of another CSD showing another channel configuration according to the present disclosure.
Fig. 15 is a truncated top view of components of another CSD showing another channel configuration according to the present disclosure.
Fig. 16 is a top view of components of another CSD showing another slot configuration according to the present disclosure.
Fig. 17 is a schematic top view of another channel configuration according to the present disclosure.
Fig. 18 is a schematic top view of another channel configuration according to the present disclosure.
Fig. 19 is a schematic top view of another channel configuration according to the present disclosure.
Fig. 20 is a top view of a component of another CSD showing another channel configuration and another slot configuration according to the present disclosure.
Fig. 21 is a block diagram describing another method for using a CSD according to the present disclosure.

### DETAILED DESCRIPTION

In the following detailed description, numerous specific details are set forth by way of examples in order to provide a thorough understanding of the relevant teachings. It will be understood that such examples are non-limiting. Numerous variations, changes, substitutions and combinations will occur to those skilled in the art without departing from the scope of the present disclosure and its teachings, and are part of the present disclosure. This includes a substitution of a feature shown in one example with a feature shown in another example, or a combination of a feature shown in one example with a feature shown in another example. All substitutions and combinations are considered part of this written description.

CSDs according to the present disclosure can feature one or more inertial dampers to fix the position of catheter tubes and prevent them from sliding or migrating over time. The phrase "inertial damper", as used herein, means a body that redirects kinetic energy from force applied to a catheter tube and: (1) converts that kinetic energy into heat energy due to friction and (2) absorbs at least some of the force by plastic or elastic deformation of the body. The phrase "inertial damping", as used herein, means the process of redirecting kinetic energy from force applied to a catheter tube and: (1) converting that kinetic energy into heat energy due to friction and (2) absorbing at least some of the force by plastic or elastic deformation of a body.

Inertial dampers according to the present disclosure can secure catheter tubes against migration without pinching the tubes. Inertial dampers can be formed of, or include components formed of, resilient materials that are plastically or elastically deformable. Suitable resilient materials can include, but are not limited to, polyethylene foam, thermoplastic elastomers and silicone.

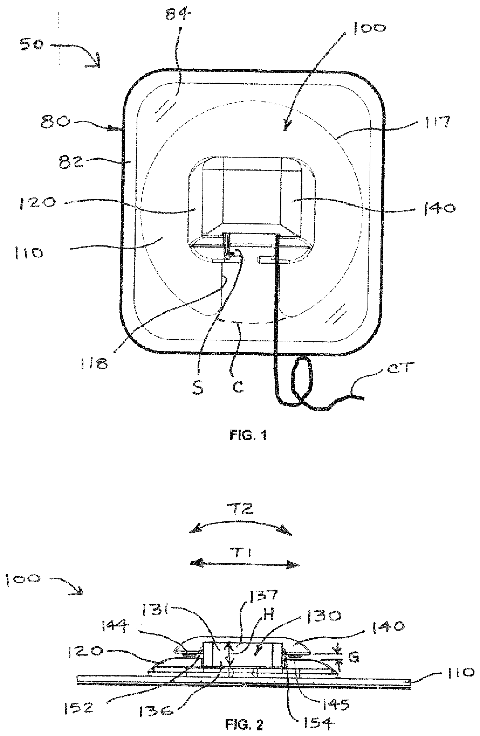

Referring to Figs. 1-3, a CSD 100 is shown according to one example, with a catheter tube CT secured to the CSD. CSD 100 includes a pad 110 having a bottom surface 111 for attachment to a patient's skin around a catheter insertion site S. Pad 110 also has a top surface 112 opposite the bottom surface 111 that faces away from the patient's skin when the CSD is attached to the patient. A first adhesive 113 is applied to bottom surface 111 for securing pad 110 to the patient's skin. A peelable cover or liner 114 is removably attached to bottom surface 111 to cover first adhesive 113 until the CSD is attached to the patient. Liner 114 includes a first liner section 115 and a second liner section 116 that are separately removable from bottom surface 111 to expose the first adhesive 113 in sections. Pads according to the present disclosure can be formed of any suitable material. In the present non-limiting example, pad 110 is formed of a 1/32 in. thick piece of polyethylene foam, medical grade, with a single coat of adhesive.

Pads according to the present disclosure can have various shapes and geometries. In the present example, pad 110 has a perimeter 117 that conforms to the shape of a circle C. One section of perimeter 117 is cut away, forming a notch 118 that extends radially from the perimeter of circle C toward the center of the circle as shown. An arc segment of circle C that crosses the peripheral opening formed by notch 118 is shown in dashed line in Fig. 1.

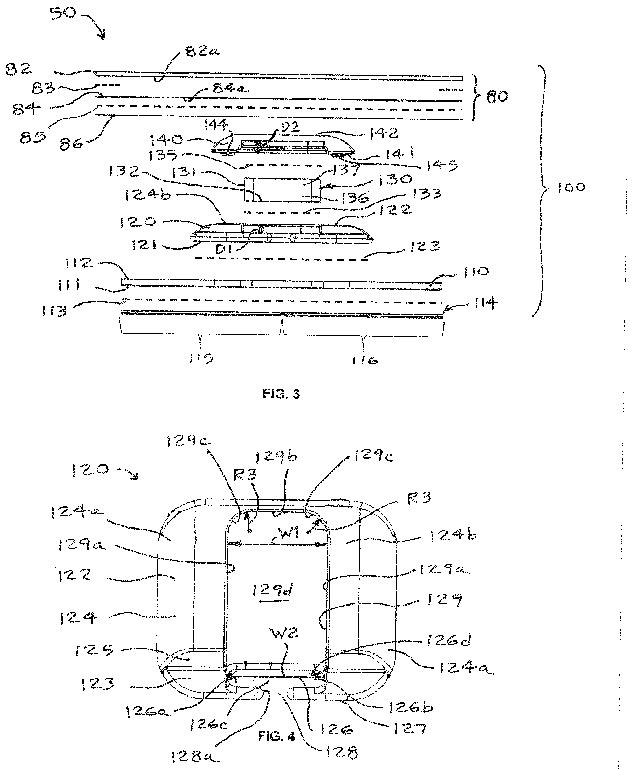

A base 120 is attached to top surface 112 of pad 110. Base 120 has a bottom surface 121 and a top surface 122 opposite the bottom surface. Bottom surface 121 of base 120 is attachable to top surface 112 of pad 110 with a second adhesive 123, which can be applied to the bottom surface of the base, the top surface of the pad, or both. Referring to Fig. 4, top surface 122 has a flat section 123 and a raised section 124 separated from the flat section by an incline 125. The thickness of flat section 123 is significantly less than raised section 124. An elongated slot 126 is defined through base 120 in flat section 123. Slot 126 has a first rounded end 126a and a second rounded end 126b that are each enclosed within flat section 123. A midsection 126c of slot 126 connects to the outermost perimeter 127 of base 120 through a catheter tube guide opening 128. Guide opening 128 has rounded edges 128a that are free of sharp corners, forming a smooth continuous transition between outer perimeter 127 and the interior wall 126d of slot 126.

Raised section 124 and incline 125 define a first receptacle 129. First receptacle 129 is generally rectangular with two opposing sidewalls 129a, one end wall 129b, and rounded corners 129c that connect the sidewalls with the end wall. First receptacle 129 also defines a first bearing face 129d. Opposing sidewalls 129a are spaced apart by a width W1 that is equal to or substantially equal to a width W2 of slot 126. Raised section 124 has curved shoulder portions 124a that extend upwardly from bottom surface 121 and level off to form a flat top deck 124b. Top deck 124b surrounds first receptacle 129 on three sides, such that it borders the sidewalls 129a and end wall 129b.

Base 120 is attachable to pad 110 such that slot 126 and guide opening 128 are aligned with a portion of notch 118. This allows CSD 100 to be maneuvered around catheter tube CT while guiding the catheter tube into slot 126 at the location where the catheter tube exits the insertion site S. Bases according to the present disclosure can be formed of any suitable material. In the present non-limiting example, base 120 is an injection molded component comprised of nylon.

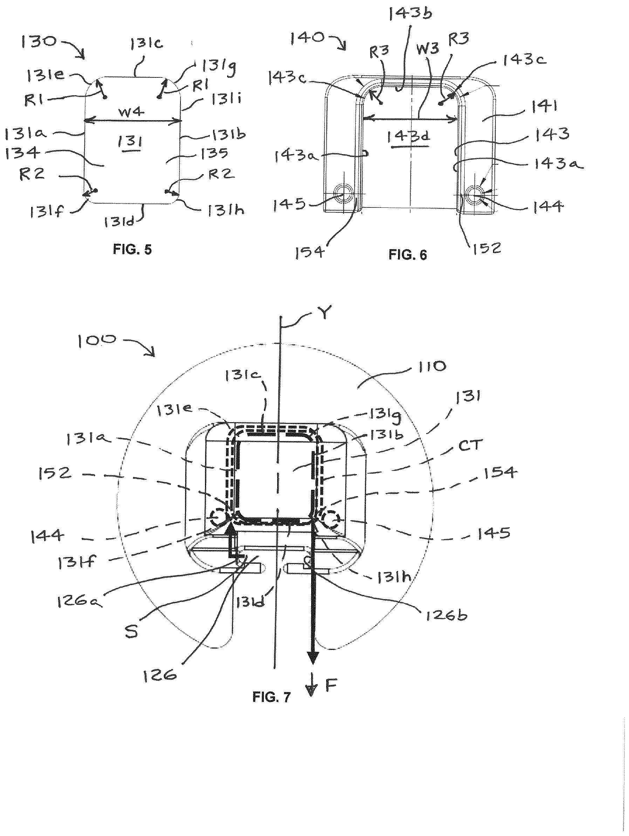

An inertial damper 130 is seated in first receptacle 129 on top of first bearing face 129d. In the present example, inertial damper 130 consists of a block 131 made of a resilient foam material and having a generally rectangular geometry. Referring to Fig. 5, foam block 131 has a first sidewall 131a, a second sidewall 131b opposite the first sidewall, a first end wall 131c and a second end wall 131d opposite the first end wall. First sidewall 131a connects to first end wall 131c at a first rounded corner 131e, and connects to second end wall 131d at a second rounded corner 131f. Second sidewall 131b connects to first end wall 131c at a third rounded corner 131g, and connects to fourth end wall 131d at a fourth rounded corner 131h. In this arrangement, the first and second sidewalls 131a, 131b, the first and second end walls 131c, 131d and the rounded corners 131e-131h form a smooth continuous perimeter wall 131i that is free of sharp corners. First and third rounded corners 131e, 131g have a first radius of curvature R1, and second and fourth rounded corners 131f, 131h have a second radius of curvature R2. R1 is greater than R2 by a factor of two. Foam blocks according to the present disclosure can be formed of any suitable foam material. In the present non-limiting example, foam block 131 is a 1/8 in. thick block of polyethylene foam.

Referring again to Figs. 2 and 3, a cover 140 rests on top of foam block 131. Foam block 131 is sandwiched between base 120 and cover 140, and supports the cover above the base in a floating manner. Cover 140 has a bottom surface 141 and a top surface 142 opposite the bottom surface. Referring to Fig. 6, bottom surface 141 defines a second receptacle 143. Second receptacle 143 is generally rectangular with two opposing sidewalls 143a, one end wall 143b and rounded corners 143c. Second receptacle 143 also defines a second bearing face 143d. Opposing sidewalls 143a are spaced apart by a width W3 that is equal to or substantially equal to a width W1 of first receptacle 129. Foam block 131 has a width W4 equal to or slightly less than width W1 of first receptacle 129 and width W3 of second receptacle 143. These dimensional relationships permit the lower portion 136 of foam block 131 to fit snugly in first receptacle 129 and the upper portion 137 of the foam block to fit snugly in second receptacle 143. Covers according to the present disclosure can be formed of any suitable material. In the present non-limiting example, cover 140 is an injection molded component comprised of nylon, like base 120.

Foam blocks according to the present disclosure can have geometries that correspond to the geometries of the receptacles in the base and cover. In the present example, rounded corners 129c of first receptacle 129 and rounded corners 143c of second receptacle 143 have a radius of curvature R3. R3 is equal to or slightly greater than first radius of curvature R1 on foam block 131. That is, R3 is equal to or slightly greater than the radius of the first and third rounded corners 131e, 131g. This dimensional relationship further permits foam block 131 to fit snugly in first and second receptacles 129, 143.

Foam blocks according to the present disclosure can be secured between the base and cover in a number of ways. Referring back to Fig. 3, foam block 131 has a bottom surface 132 that attaches to first bearing face 129d with a third adhesive 133. Foam block 131 also has a top surface 134 that attaches to second bearing face 143d with a fourth adhesive 135. Third adhesive 133 and fourth adhesive 135 are applied as coatings on the bottom surface 132 and top surface 134 of foam block 131, respectively. It will be appreciated, however, that third adhesive 133 and fourth adhesive 135 can be applied as coatings on the first bearing face 129d and second bearing face 143d, respectively, which can be done as an alternative to coating foam block 131, or in addition to coating the foam block.

Foam block 131 is sandwiched between base 120 and cover 140 with lower portion 136 received and seated in first receptacle 129 and upper portion 137 received and seated in second receptacle 143. Referring to Figs. 2 and 3, first receptacle 129 has a first depth D1 and second receptacle 143 has a second depth D2. Foam block 131 has a height H that is greater than the sum of the first depth D1 and second depth D2. As such, foam block 131 separates base 120 and cover 140 by a small gap G.

First portion 136 is seated in first receptacle 129 in a snug fit between sidewalls 129a, and second portion 137 is seated in second receptacle 143 in a snug fit between sidewalls 143a. Sidewalls 129a, sidewalls 143a, third adhesive 133, and fourth adhesive 135 prevent foam block 131 from translating in the first and second receptacles 129, 143. That is, bottom surface 132 remains fixed relative to first bearing face 129d, and top surface 134 remains fixed relative to second bearing face 143d. Despite these fixations, base 120 and cover 140 are movable in translation relative to one another in lateral directions due to the deformability of foam block 131. The deformability of foam block 131 also allows cover 140 to tilt in multiple directions relative to base 120. Fig. 2 shows two possible directions of translation with arrows T1 and two possible directions of tilting with arrows T2. Arrows T1 and T2 illustrate directions of translation within the plane of the Figure and directions of tilting relative to an axis normal to the plane of the Figure. Cover 104 is also translatable and tiltable relative to other planes and axes.

CSDs according to the present disclosure can have one or more retaining members that serve multiple functions. Retaining members are useful for limiting the tilting of the cover relative to the base and maintaining a minimum clearance between bottom surface 141 of cover 140 and top surface 122 of base 120. This reduces the potential for pinching of catheter tube CT between bottom surface 141 of cover and top surface 122 of base 120. Retaining members also provide a coil management structure that keeps catheter tube CT closely wound around inertial damper 130, as will be explained. Retaining members can have a variety of configurations and arrangements between the base and cover. In addition, retaining members can be placed on the base only, the cover only, or both the base and cover.

In the present example, two retaining members are provided in the form of a first post 144 and a second post 145, as shown in Figs. 2, 3 and 6. First and second posts 144, 145 project from bottom surface 141 of cover 140 toward top surface 122 of base 120 when the parts are assembled. First post 144 is positioned to align with and abut top deck 124b on one side of first receptacle 129, and second post 145 is positioned to align with and abut the top deck on the opposite side of the first receptacle. First post 144 and second post 145 each limit how far cover 140 can be laterally tilted so as to maintain a minimum spacing between the bottom surface 141 and top surface 122. The minimum spacing can be selected to be larger than the diameter of the largest catheter tube CT on the market, incorporating a factor safety to prevent impingement of catheter tubes in all applications.

When CSD 100 is assembled, first post 144 is spaced from foam block 131 by a first lateral clearance 152. Second post 145 is spaced from foam block 131 by a second lateral clearance 154. First and second lateral clearances 152, 154 provide small spaces between first and second posts 144, 145 and foam block 131.

When CSD 100 is placed over a catheter insertion site S, gap G is adapted to receive a section of catheter tube CT that extends from slot 126 out of the insertion site. Gap G is configured to receive the section of catheter tube CT between base 120 and cover 140 and permit the catheter tube to be wound around foam block 131. Referring to Fig. 7, another top view of CSD 100 is shown to illustrate how catheter tube CT can be wound beneath cover 104. Hidden features, such as foam block 131, first and second posts 144, 145, and a portion of catheter tube CT, are shown in dashed line. The two arrow heads on CT depict the direction of winding, beginning at insertion site S, and ending at the lower right of the Figure.

CSD 100 is symmetrical with respect to an axis Y that bisects slot 126. Therefore, slot 126, first and second posts 144, 145, foam block 131, and first and second clearances 152, 154 are arranged symmetrically with respect to axis Y. This symmetry allows the user to place CSD 100 over catheter insertion site S with catheter tube CT positioned within either first rounded end 126a of slot 126 or second rounded end 126b. This symmetry further permits catheter tube CT to enter gap G on either side of axis Y. Moreover, this symmetry permits the user to wind catheter tube CT around foam block 131 in either a clockwise direction or a counterclockwise direction. Therefore, the symmetrical arrangement allows the user to choose the relative position of CSD 100 over insertion site S according to preference, and choose the direction of winding according to preference.

CSD 100 is shown in Fig. 7 as being positioned over insertion site S with catheter tube CT positioned in first rounded end 126a of slot 126. From this location, catheter tube CT enters gap G on the left side of axis Y and is fed through first clearance 152. Catheter tube CT extends further through gap G, where it extends along first sidewall 131a. Catheter tube CT then continues around first rounded corner 131e, first end wall 131c, third rounded corner 131g, and second sidewall 131b before entering second clearance 154. From second clearance 154, catheter tube CT extends further along fourth rounded corner 131h, second end wall 131d and second rounded corner 131f until it returns to first sidewall 131a. As such, catheter tube CT is wound around foam block 131 in a clockwise direction, forming a complete loop around the perimeter of foam block 131. Catheter tube CT is looped again around first sidewall 131a, first rounded corner 131e, first end wall 131c, third rounded corner 131g, second sidewall 131b and through second clearance 154, and then exits gap G on the right side of axis Y.

Catheter tube CT bears against and frictionally engages some or all of the sidewalls 131a, 131b, end walls 131c, 131d, and rounded corners 131e-131h when the catheter tube is wound around foam block 131. The frictional engagement(s) between catheter tube CT and foam block 131 allows the foam block to convert kinetic energy in catheter tube CT (i.e. energy from tensile force applied to the catheter tube) into heat energy due to frictional resistance. Foam block 131 also absorbs some of the force applied to catheter tube CT by plastically or elastically deforming. For example, foam block 131 can buckle, bend and/or twist in response to a force applied to catheter tube CT, absorbing some of the kinetic energy as the foam changes shape.

CSDs according to the present disclosure are configured to be covered by bio-occlusive dressings to reduce the risk of infection at the catheter site. CSD components have geometries that allow dressings to be applied easily over the device and firmly adhere to the patient. The covers and bases can have a very low height to width ratio, resulting in a low profile on the patient's skin. The low profile limits how much the device projects above the skin surface and consequently reduces the required size of the dressing. Referring to Fig. 2, for example, base 120, block 131 and cover 140 have a height to width ratio of approximately 1:5 when assembled. It will be appreciated that higher ratios, for example 1:4 or 1:3, can also be used, but will project further out from the patient and may require larger dressings.

CSDs according to the present disclosure also have geometries that allow dressings to lie flat against the device components and the patient. In the present example, base 120 and cover 140 form a pyramidal geometry in the assembled state, with a rectangular base and sloped sides that taper inwardly as the sides extend from the base to the cover. The sloped faces on base 120 are generally aligned with the sloped faces on cover 140. In addition, changes in contour along the sloped faces are rounded, defining smooth compound curvatures. This geometry allows dressings to be easily applied over CSD 100 and pressed flat against the cover and base without catching on sharp edges.

A variety of dressings can be applied over CSD 100. Dressings can be supplied with CSD 100 in a kit. Alternatively, CSD 100 can be packaged as a standalone device, and dressings can be provided separately. Figs. 1 and 3 show a kit 50 that includes the CSD 100 and a bio-occlusive dressing 80 that is packaged with the CSD. Dressing 80 includes a foam border 82 and a transparent window 84. Foam border 82 is formed of a 1/16 in. thick piece of medical grade polyethylene foam. Window 84 is made of a medical grade polyurethane film. Foam border 82 is attached to window 84 by a fifth adhesive 83 applied to a patient-facing side 82a of the foam border. Window 84 has a sixth adhesive 85 applied to a patient-facing side 84a of the window to attach dressing 80 to CSD 100 and the patient. Dressing 80 also has a release liner 86 applied to patient-facing side 84a of window 84 to cover sixth adhesive 85 until the dressing is ready to be used. The total area of dressing 80 is larger than the upper surface area of CSD 100, allowing window 84 to cover the entire footprint of the CSD with all components visible through the window as shown.

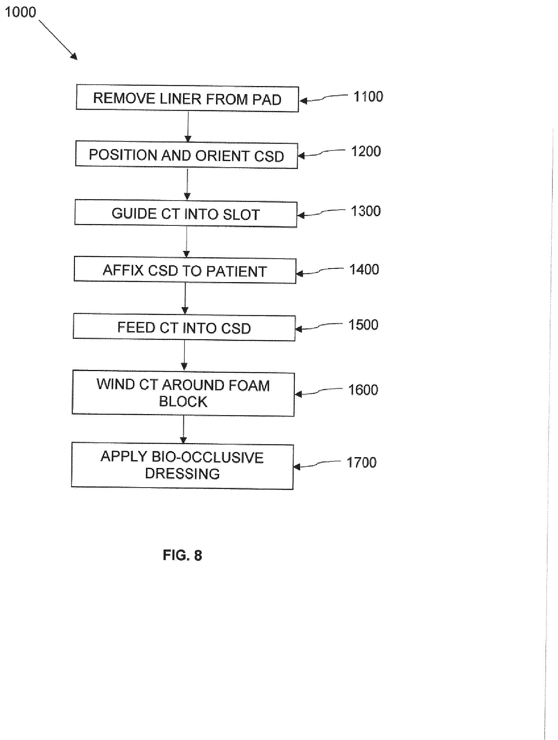

Fig. 8 illustrates one possible method 1000 for using a CSD according to the present disclosure. The following section describes steps of method 1000 assuming that CSD 100 is used. It will be appreciated that the following steps can be used with other CSDs having similar designs to CSD 100, and are not exclusively limited to CSD 100. The following section further assumes that the catheter insertion site S is prepared according to appropriate protocol before performing the described steps.

In step 1100, first and second liner sections 115, 116 are removed from pad 110 to expose first adhesive 113 on bottom surface 111 of the pad.

In step 1200, CSD 100 is positioned over insertion site S and rotated to the proper orientation. The user grips one side of pad 110 with a first hand (either left hand or right hand) and holds CSD 100 so that notch 118 opens or faces toward the user. This orientation is represented in Fig. 1. CSD 100 is then moved to a position in which notch 118 is in proximity to catheter tube CT.

In step 1300, the catheter tube CT is carefully guided into slot 126 of CSD 100. This step is preferably performed without allowing bottom surface 111 of pad 110 and first adhesive 113 to contact the patient. With their first hand still gripping pad 110, the user grips catheter tube CT with their other hand and raises the catheter tube if needed so that the catheter tube does not lie flat on the patient. The user maneuvers CSD 100 around the catheter tube so that the catheter tube is received into notch 118. The user then maneuvers CSD 100 further to align a section of the catheter tube CT with guide opening 128. When alignment is achieved, the user adjusts the position of CSD 100, the catheter tube CT, or both, to guide catheter tube CT into slot 126. Base 120 has a smooth front edge 120a without sharp corners that extends on each side of guide opening 128. If catheter tube CT misses guide opening 128 and contacts the front edge 120a of base 120 during the maneuvering process, the catheter tube will slide along the front edge until it reaches one of the rounded edges of the guide opening. Rounded edges 128a are configured to draw catheter tube CT into slot 126 without encountering any sharp edges or obstructions. Once catheter tube CT enters slot 126, the user can fine tune the relative positions of CSD 100 and catheter tube CT so that the catheter tube is located in first rounded end 126a or second rounded end 126b, according to preference. For example, if the user prefers winding catheter tube CT around foam block 131 in a clockwise direction, as shown in Fig. 6, the user can position the catheter tube in first rounded end 126a so that the winding begins on the left side of axis Y. If the user prefers winding catheter tube CT around foam block 131 in a counterclockwise direction, the user can position the catheter tube in second rounded end 126b so that the winding begins on the right side of axis Y.

In step 1400, the user affixes CSD 100 to the patient around insertion site S. The user tilts pad 110 if needed so that bottom surface 111 of pad 110 is normal to an imaginary line extending vertically from insertion site S. The user then lowers pad 110 onto the patient's skin and presses the pad down around the insertion site S so that first adhesive 113 forms a bond with the patient's skin.

In step 1500, the user feeds catheter tube CT into CSD 100. The user grips a section of catheter tube CT close to insertion site S and carefully inserts the tube section into gap G between base 120 and cover 140. The user can do this by gently pulling on the catheter tube CT to apply a small amount of tension so that the catheter tube section is straight. The straight length of tube is then positioned either on the left side or right side of base 120 and cover 140 (depending on preference) with the straight length of tube generally aligned with gap G. Upon achieving this alignment, the user guides catheter tube CT into gap G. The user continues to apply a small amount of tension to catheter tube CT so that it snaps into gap G and bears against foam block 131. Depending on design preferences and dimensions, first post 144 or second post 145 may create a small obstruction that partially blocks the path of catheter tube CT as it snaps into the first clearance 152 or second clearance 154, respectively. Therefore, first and second posts 144 and 145 preferably have rounded ends that allow catheter tube CT to slide smoothly around the ends of the posts. Upon reaching foam block 131, catheter tube CT will be disposed in first clearance 152 if entering from the left side or second clearance 154 if entering from the right side.

In step 1600, the user winds catheter tube CT around foam block 131. Catheter tube CT can be wound in a clockwise direction in the manner described previously. For example, the user can insert catheter tube CT into gap G from the left side of base 120 and cover 140, and then work around the top, right side, and bottom of the base and cover, as shown in Fig. 7. Alternatively, catheter tube CT can be wound in a counterclockwise direction, in which case the user inserts catheter tube CT into gap G from the right side of base 120 and cover 140, and then works around the top, left side, and bottom of the base and cover. The user may need to pull gently on catheter tube CT each time the catheter tube enters the left and right sides and encounters the first post 144 and second post 145, so as to cause the catheter tube to slip beneath the posts and snap into the first and second clearances 152, 154. Catheter tube CT can be wound around foam block 131 one or more times to secure the catheter tube in CSD 100. A small amount of tension can be maintained on catheter tube CT throughout the winding process to keep the catheter tube coil snug around foam block 131. In the event that some tension in catheter tube CT is lost, the coil may loosen a small amount, such that one or more sections of the loop(s) move away from foam block 131. In such a case, first and second posts 144, 145 serve as coil retaining members that limit the amount of unraveling of the coil, so that the coil is maintained in partial engagement with and close proximity to foam block 131.

During the winding process, cover 140 can be tilted side-to-side, or laterally, to increase the height of gap G on one side of CSD 100. This may be desirable to make it easier to fit catheter tube CT into gap G. To increase the height of gap G on the left side of CSD 100, for example, the user presses lightly on the right side of cover 140 to raise the left side of the cover relative to the base. To increase the height of gap G on the right side of CSD 100, the user presses lightly on the left side of cover 140 to raise the right side of the cover relative to the base. When tilting cover 140 to the right, second post 145 limits how much the cover is tilted to reduce the potential for pinching catheter tube CT. Similarly, when tilting cover 140 to the left, first post 144 limits how much the cover is tilted to also reduce the potential for pinching catheter tube CT.

Once catheter tube CT is wound around foam block 131, the catheter tube is secured against movement by inertial damping. Catheter tube CT bears against and frictionally engages at least some of the sidewalls 131a, 131b, end walls 131c, 131d, and rounded corners 131e-131h of foam block 131. This allows foam block 131 to convert kinetic energy from force applied to catheter tube CT into heat energy as a result of frictional resistance between the catheter tube and foam block. Foam block 131 also absorbs some of the force applied to catheter tube CT by plastically or elastically deforming.

Inertial damping can be explained further with reference to Fig. 7 and a hypothetical tensile force F applied to catheter tube CT. Force F could be caused by accidental contact between catheter tube CT and its surroundings, such as a garment or article of clothing that pulls on the catheter tube as the patient moves. Force F is transmitted along the length of catheter tube CT to the coiled section(s) of tube around foam block 131, where the coiled section(s) is placed under increased tension. During this period of increased tension, at least some of the coils tighten and press against foam block 131, creating additional frictional resistance. Frictional resistance limits or prevents catheter tube CT from sliding against foam block 131. The sidewalls, end walls and rounded corners of foam block 131 are radially compressible in response to tightening of the coils, causing plastic or elastic deformation that absorbs some of the energy and stores some of the energy as potential energy in the foam. When the tensile force is released, i.e. when the pulled section of catheter becomes free from the garment that is pulling on the tube, some or all of the tension in the coil(s) can also be released. In such a case, the energy stored in foam block 131 is released, allowing foam block 131 to expand and return back to or toward its original shape. The tensile force along the length of the tube dissipates through a combination of energy conversion and absorption by foam block 131. Consequently, foam block 131 is configured to convert and absorb energy applied to the section of catheter tube CT that is external to the patient to prevent that energy from displacing the section of catheter tube CT that is internal to the patient.

Referring again to Fig. 8, the method can further include the step of applying a bio-occlusive dressing in step 1700. For example, the previously-described dressing 80 can be applied over CSD 100. The user can hold dressing 80 above CSD 100 with one hand, with the patient-facing side 84a and release liner 86 facing toward the CSD. The user then removes release liner 86 with the other hand. After release liner 86 is removed, the user positions window 84 above CSD 100 with the center of the CSD centered in the window. The user then applies dressing 80 onto the patient by pressing down on the edges of the dressing so that sixth adhesive 85 adheres to the patient. Window 84 can also be pressed onto CSD 100 and catheter tube CT so that sixth adhesive 85 attaches to the CSD and catheter tube. Dressing 80 covers the entire footprint of CSD 100, providing a protective seal around the CSD to seal the insertion site S from moisture and reduce the risk of infection at the insertion site.

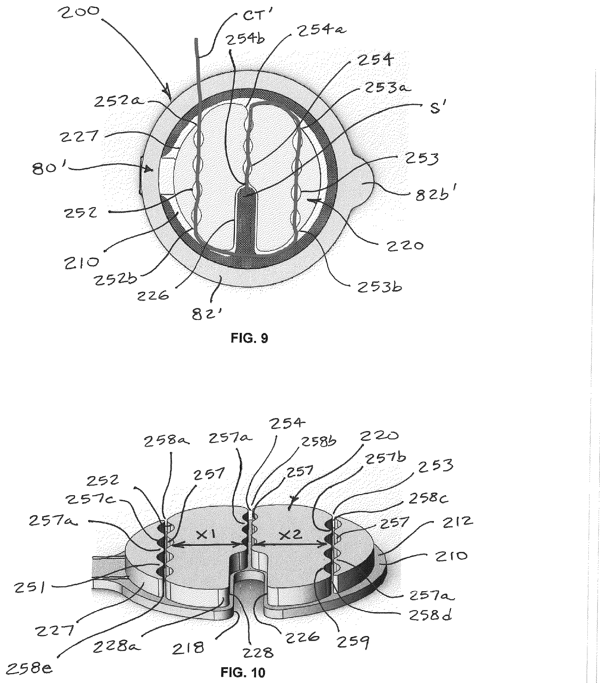

Referring next to Figs. 9-11, a CSD 200 is shown according to another example, with a catheter tube CT' secured to the CSD. CSD 200 includes a base in the form of a pad 210 having a bottom surface 211 for attachment to a patient's skin around a catheter insertion site S'. Pad 210 also has a top surface 212 opposite the bottom surface 211 that faces away from the patient's skin when the CSD is attached to the patient. A first adhesive 213 is applied to bottom surface 211 for securing pad 210 to the patient's skin. A peelable cover or liner 214 is removably attached to bottom surface 211 to cover first adhesive 213 until the CSD is used. Liner 214 is removable from bottom surface 211 to expose the first adhesive 213. Pads according to the present disclosure can be formed of any suitable material. In the present non-limiting example, pad 210 is formed of a 1/32 in. thick piece of polyethylene foam, medical grade, with a single coat of adhesive, similar to the previous example.

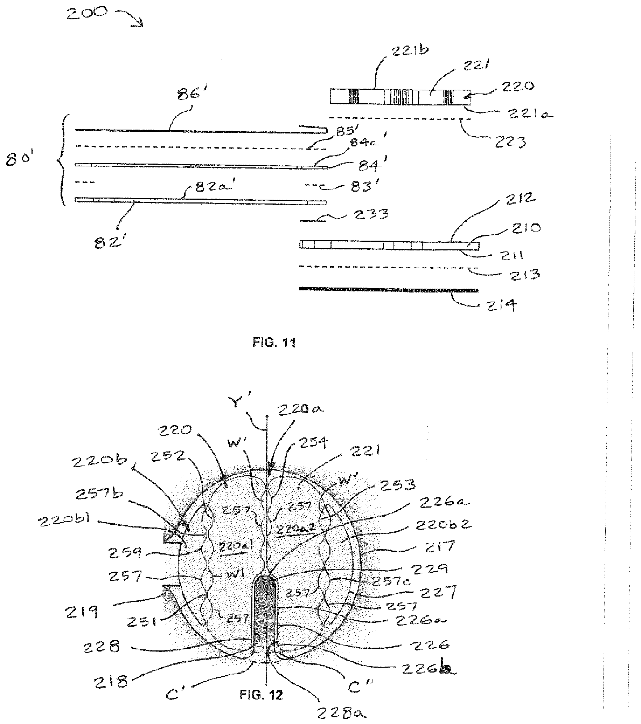

Pads according to the present disclosure can have various shapes and geometries. Referring to Fig. 12, pad 210 has a perimeter 217 that conforms to the shape of a circle C'. One section of perimeter 217 is cut away, forming a notch 218 that extends radially from the perimeter of the circle toward the center of the circle as shown. An arc segment of circle C' that crosses the peripheral opening formed by notch 218 is shown in dashed line. Pad 210 also includes an extension portion 219 that projects from perimeter 217.

An inertial damper 220 is attached to top surface 212 of pad 210. Inertial damper 220 has a foam body 221 with a bottom surface 221a and a top surface 221b opposite the bottom surface. Bottom surface 221a of foam body 221 is attachable to top surface 212 of pad 210 with a second adhesive 223, which can be applied to the bottom surface of the base, the top surface of the pad, or both. Foam body 221 has a perimeter 227 that conforms to the shape of a circle C". An arc segment of circle C" that crosses the peripheral opening formed by notch 218 is shown in dashed line in Fig. 12.

An elongated slot 226 is defined through foam body 221. Slot 226 has a first end 226a that lies adjacent to the center of circle C" and a second end 226b that opens out through perimeter 227. First end 226a has a semi-circular or rounded shape free of sharp corners, and is configured to guide catheter tube CT' from insertion site S' into inertial damper 220. Second end 226b forms a catheter tube guide opening 228 with rounded edges 228a that are free of sharp corners, forming a smooth continuous transition between outer perimeter 227 and interior wall 226d of slot 226. Catheter tube guide opening 228 and rounded edges 228a assist in allowing catheter tube CT' to be received in slot 226 as CSD 200 is placed around the catheter tube and insertion site S'.

Foam body 221 is attachable to pad 210 such that slot 226 and guide opening 228 are aligned with notch 218. This aligned arrangement allows CSD 200 to be maneuvered around catheter tube CT' while guiding the catheter tube into slot 226 at the location where the catheter tube exits the insertion site S'. Notch 218 and slot 226 also form an unobstructed viewing aperture 229 that allows the caregiver to see insertion site S' during and after placement of CSD 200.

Foam body 221 is attached to pad 210 with circle C" arranged concentrically to circle C', and with slot 226 aligned with notch 218, as shown. Bases according to the present disclosure can be formed of any suitable material. In addition, bases according to the present disclosure can be formed in one or more sections. In the present non-limiting example, foam body 221 is formed of 1/8 in. thick polyethylene foam coated with second adhesive 223 and divided into four separate sections.

The four sections of foam body 221 are arranged relative to one another on pad 210 to collectively fit within circle C", with each section attached to the pad with second adhesive 223. The sections are spaced relative to one another and arranged to allow catheter tube CT' to be wound around foam body 221 and looped through the foam body. Inertial dampers according to this embodiment can feature any number of sections to facilitate winding and looping of a catheter tube. In addition, inertial damper sections can have geometries and relative spacings to create a symmetrical arrangement of inertial damper sections or an asymmetrical arrangement of inertial damper sections. For example, the inertial damper can have at least two outside or "lateral" damper sections having the same shape and top surface area arranged in a mirror arrangement, or at least two lateral damper sections having a different shape and top surface area. In addition, or in the alternative, the inertial damper can have at least two inside or "medial" damper sections having the same shape and top surface area arranged in a mirror arrangement, or at least two medial damper sections having a different shape and top surface area. A symmetrical arrangement of damper sections can provide a more even distribution of force on foam body 221, but an asymmetrical arrangement can allow more foam material to be placed on a particular side or in a particular section that is expected to receive more force from catheter tube CT' than other areas.

In the present example, inertial damper 220 consists of a medial inertial damper 220a that spans an interior area of circle C" and a lateral inertial damper 220b that spans two lateral areas of circle C". Medial inertial damper 220a consists of a first medial inertial damper 220a1, shown to the left of slot 226, and a second medial inertial damper 220a2, shown to the right of the slot. Lateral inertial damper 220b consists of a first lateral inertial damper 220b1, shown to the left of first medial inertial damper 220a1, and a second lateral inertial damper 220b2, shown to the right of second medial inertial damper 220a2. First medial inertial damper 220a1 and second medial inertial damper 220a2 are symmetrically arranged relative to an axis Y' that crosses the diameter of circle C" and bisects slot 226. Similarly, first lateral inertial damper 220a1 and second lateral inertial damper 220a2 are symmetrically arranged relative to axis Y'.

First lateral inertial damper 220b1 and first medial inertial damper 220a1 are separated by a first channel 252. Second lateral inertial damper 220b2 and second medial inertial damper 220a2 are separated by a second channel 253. First medial inertial damper 220a1 and second medial inertial damper 220a2 are separated by a third channel 254. First channel 252, second channel 253 and third channel 254 extend parallel to one another through foam body 221.

First channel 252, second channel 253 and third channel 254 each have a channel width W' adapted to receive a section of catheter tube CT' in one or more passes. Referring again to Fig. 9, first channel 252 has a first end 252a and a second end 252b opposite the first end. First end 252a and second end 252b are each open to perimeter 227 of foam body 221. Likewise, second channel 253 has a first end 253a and a second end 253b that are each open to perimeter 227. This arrangement allows catheter tube CT' to be inserted into first channel 252 from two different peripheral locations of foam body 221 and/or inserted into second channel 253 from two different peripheral locations of the foam body.

Third channel 254 has a first end 254a open to perimeter 227 and a second end 254b that intersects slot 226. This arrangement allows a section of catheter tube CT' that protrudes from insertion site S' to be fed through second end 254b and into third channel 254. Consequently, first channel 252, second channel 253 and third channel 254 allow catheter tube CT' to be fed from insertion site S' into third channel 254, looped around the outside of one or more of the medial inertial dampers 220a1, 220a2 and lateral inertial dampers 220b1, 220b2, inserted through one or more of the first and second channels 252, 253, and if desired, looped again around the outside of one or more the medial inertial dampers and lateral inertial dampers.

Referring to Fig. 10, first channel 252 is spaced from third channel 254 by a first spacing X1, and second channel 253 is spaced from third channel 254 by a second spacing X2. First spacing X1 is equal to second spacing X2, so that first channel 252 and second channel 253 are equidistant to third channel 254. First, second and third channels 252-254 open out to perimeter 227 at five locations on the perimeter, forming openings 258a-258e that are arranged in a circular pattern conforming to circle C". The distribution of openings 258a-258e around perimeter allows the user to wind catheter tube CT' around the outside of the foam body 221 in a clockwise direction or a counterclockwise direction according to preference. The distribution of openings 258a-258e also allows the user to coil the tube partially around or completely around any of the medial inertial dampers 220a1, 220a2 and/or lateral inertial dampers 220b1, 220b2 according to preference. Catheter tube CT' can be coiled around the perimeter of any one the medial inertial dampers 220a1, 220a2 and/or lateral inertial dampers 220b1, 220b2, and/or be coiled around the combined perimeter of two or more of the medial inertial dampers 220a1, 220a2 and/or lateral inertial dampers 220b1, 220b2.

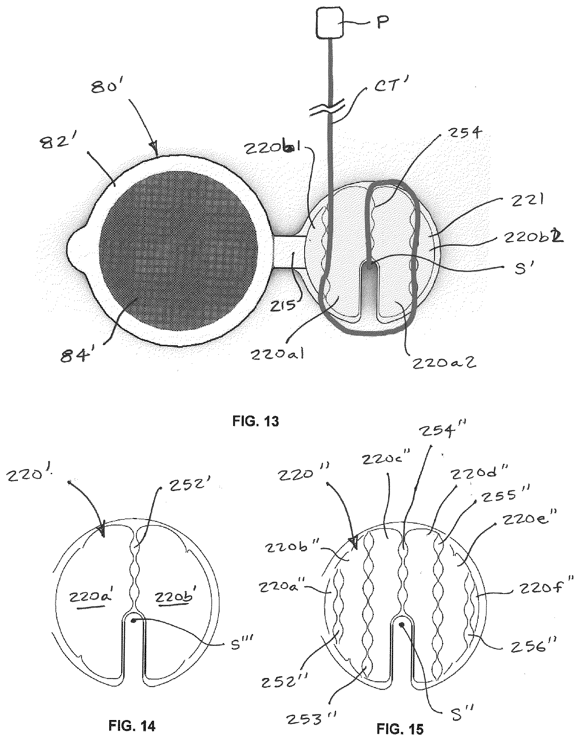

Referring to Fig. 13, catheter tube CT' is shown extending from insertion site S' and through foam body 221 in a circumvoluted manner. Catheter tube CT' is initially passed through third channel 254 so that the tube emerges from the third channel at opening 258b. Catheter tube CT' is then wound in a clockwise direction around an outer edge of second medial inertial damper 220a2 and through second channel 253 so that the tube emerges from the second channel at opening 258d. From opening 258d, catheter tube CT' is wound further in a clockwise direction around an outer edge of second medial insert damper 220a2, an outer edge of first medial inertial damper 220a1 and through first channel 252 so that the tube emerges from the first channel at opening 258a. From there, catheter tube CT' extends to a source of fluid to be administered to the patient. In Fig. 13, the source is schematically shown as an infusion pump P connected to catheter tube CT'. First lateral inertial damper 220b1 and second lateral inertial damper 220b2 act as coil retaining members, similar to posts 144, 145 in the first example, so that the loops remain closely wound around first and second medial inertial dampers 220a1, 220a2.

The tube-securing channels according to the present disclosure can have one or more sidewall configurations to stabilize and secure a catheter tube in the inertial damper. Accordingly, foam bodies according to the present disclosure can be cut in a number of ways to form different sidewall configurations. For example, the channel or channels can have flat continuous sidewalls with a uniform width between the sidewalls that is smaller than the diameter of tubes to be secured. Flat sidewalls provide a simple geometry, but they can create difficulties when inserting tubes into the inertial damper. Flat side walls tend to engage all or substantially all of a catheter tube. If a catheter tube has a diameter significantly larger than the width of the channel, then the sidewalls can create a significant amount of frictional resistance along the entire length of the tube segment that makes insertion and removal of the tube difficult.

The applicant has found that discontinuities in the sidewalls in the form of undulations strike a proper balance between: (1) providing enough surface area to secure a catheter tube against unwanted migration and (2) limiting the amount of frictional resistance encountered during insertion of tubes into the channels so that insertion is easy. The undulations can be spaced at regular intervals or irregular intervals along the length of the channel and have a variety of shapes. Suitable shapes include, but are not limited to zig-zag edges, serrations, saw teeth and wave patterns. The undulations can form a series of narrow or constricted sections and wider sections, where the narrow sections have channel widths that are slightly less than the diameter of the catheter tube CT' in order to frictionally engage areas on the exterior of the catheter tube.

Referring to Figs. 10 and 12, channels 252-254 each have a pair of undulating sidewalls 257. Each sidewall 257 has a series of undulations 257a that extend in a sinusoidal or wave-like manner. Undulations 257a are comprised of crests or convex portions 251 and troughs or concave portions 259. The undulations 257a are provided in a mirror arrangement on opposite sides of the channels. That is, the convex portions 251 are aligned with and face one another on each side of each channel, and the concave portions 259 are aligned with and face one another on each side of each channel. In this arrangement, convex portions 251 form narrow constricted sections 257b, and concave portions 259 form wider unconstricted sections 257c. The channel width in the narrow constricted sections 257b is less than diameters of common catheter tubes. For example, the channel width in the narrow constricted sections 257b can be between approximately 0.0125 in. and 0.0250 in. The channel width in the wider unconstricted sections 257c is greater than diameters of common catheter tubes. For example, the channel width in the unconstricted sections 257c can be between approximately 0.100 in. and 0.130 in. Channels 252-254 have universal channel widths in the narrow constricted sections 257b to accommodate 18G - 24G tubes.

The softness of foam body 221 allows sidewalls 257 to be pushed outwardly and expand under stored energy as catheter tube CT' is inserted into each channel. Once catheter tube CT' is inserted into a channel, the expansion of the sidewalls 257 creates reaction forces in the foam that bear against the catheter tube. The reaction forces gently squeeze or bear against the exterior of catheter tube CT' to secure the position of the tube without pinching the tube walls or altering the size or shape of the tube passage. Convex portions 251 account for only a small fraction of the total sidewall lengths. Therefore, sidewalls 257 only bear against a small percentage of the outer surface area of catheter tube CT'. Consequently, the reaction forces create only a small to moderate amount of frictional resistance that is sufficient to prevent accidental dislodging of the catheter tube CT, but not so large as to make it difficult or cumbersome to insert the catheter tube into the channel.

Sidewalls 257 in each channel do not contact one another in the present example. It will be appreciated, however, that opposing sidewalls of channels can contact one another without departing from the present disclosure and what is contemplated. For example, the constricted sections of channels can contact one another such that the channel width W' is 0 mm between the sidewalls in the constricted sections. This may be desirable for very small catheter tubes, or applications where a greater amount of frictional engagement is desired.

The ratio of the thickness of pad 210 and the diameter of circle C' is relatively small. The ratio of the thickness of foam body 221 to the diameter of circle C" is also relatively small. These ratios can be as small as 1:16 - 1:20, or smaller, for example. This arrangement allows CSD 200 to have a very low profile, so that the CSD is comfortable to wear and less prone to be caught on clothing than CSD designs with taller profiles. The small ratios and presence of multiple channels also provide an efficient securement geometry that devotes a large amount of surface area inside inertial damper 220 to convert and absorb kinetic energy, while taking up very little space above the insertion site S'.

The present disclosure contemplates other arrangements that have less than four inertial damper sections, as well as arrangements having more than four inertial damper sections. For example, the inertial damper could have just two dampers, with one to the left of axis Y' and the other to the right of axis Y'. Alternatively, the inertial damper could have six dampers, with three to the left of axis Y' and the other three to the right of axis Y'.

Fig. 14 shows one alternate configuration for an inertial damper 220' featuring two symmetrically-arranged damper sections 220a' and 220b'. Damper sections 220a' and 220b' are semi-circular shaped dampers that are separated by a single channel 252'. In this arrangement, a catheter tube can be passed from an insertion site S", through channel 252' and wound around one or both damper sections 220a' and 220b'. After completing one wind or loop around one or both damper sections 220a', 220b', the catheter tube can be fed through channel 252' in one or more subsequent passes.

Fig. 15 shows another alternate configuration for an inertial damper 220" featuring six symmetrically-arranged damper sections 220a'', 220b", 220c", 220d", 220e"and 220f". Damper sections 220a"-220f" are separated by five channels 252"-256". In this arrangement, a catheter tube can be passed from an insertion site S‴ and through foam body in a circumvoluted manner. For example, a catheter tube can be passed from an insertion site S‴ through third channel 254", pulled clockwise around an outer edge of damper section 220d", looped through fourth channel 255", pulled clockwise again around outer edges of damper sections 220d" and 220c", looped through second channel 253", pulled clockwise again around the outer edges of damper sections 220c"-220e", looped through fifth channel 256", pulled clockwise again around outer edges of damper sections 220b"-220e", and finally looped through first channel 252". The catheter tube can then be extended out and connected to an infusion pump or other source. Alternatively, the catheter tube can be wound again around one or more of damper sections 220a"-220f" and reinserted through one or more of channels 252"-256" to further secure the tube.

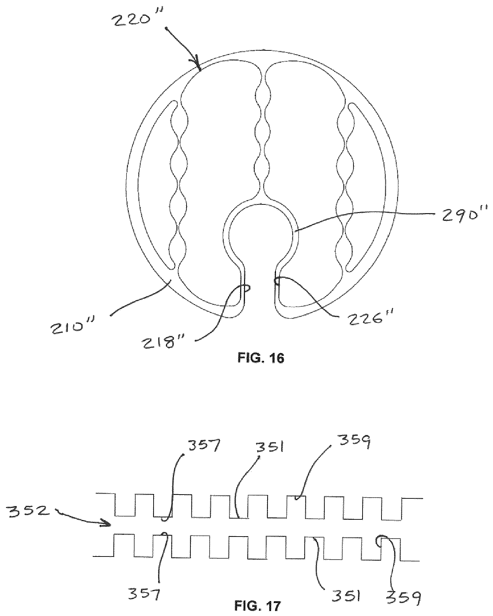

Fig. 16 shows a pad 210‴ and inertial damper 220‴ according to another configuration. Pad 210‴ has a keyhole-shaped notch 218‴, and inertial damper 220‴ has a keyhole-shaped slot 226‴. The keyhole-shaped geometries of notch 218‴ and slot 226‴ provide a bulbous shaped aperture 290‴ toward the centers of pad 210‴ and inertial damper 220‴. Aperture 290‴ provides a larger opening above the insertion site in comparison to the previous examples.

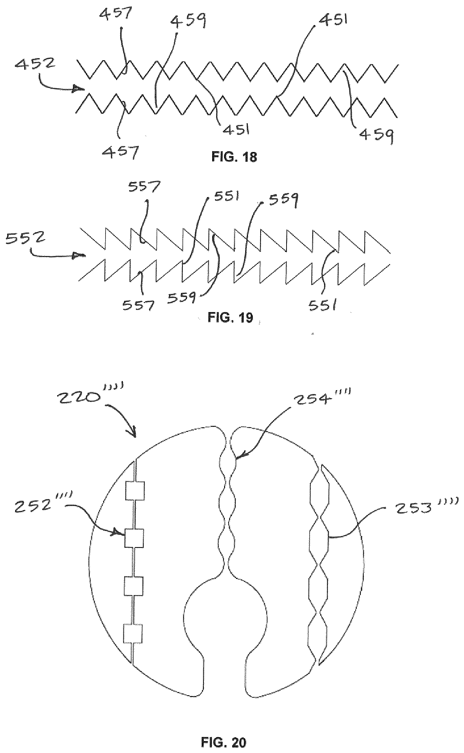

Figs. 17-19 show examples of alternative channel geometries that can be used. Fig. 17 shows a channel 352 with undulating sidewalls 357 that feature rectangular-shaped crests 351 and rectangular-shaped troughs 359. Fig. 18 shows a channel 452 with undulating sidewalls 457 that feature crests 451 and troughs 459 conforming to equilateral triangles. Fig. 19 shows a channel 552 with undulating sidewalls 557 that feature crests 551 and troughs 559 conforming to right triangles. It will be appreciated that other geometries according to the present disclosure can be used that strike a balance between providing enough frictional resistance to secure a catheter tube against unwanted migration and allowing catheter tubes to be inserted into the channel(s) easily.

Fig. 20 shows an inertial damper 220ʺʺ according to another configuration. Inertial damper 220ʺʺ has first, second and third channels 252ʺʺ-254ʺʺ with three different wall geometries. First channel 252ʺʺ has undulations in a rectangular pattern, second channel 253ʺʺ has undulations in a polygonal pattern, and third channel 254ʺʺ has undulations in a sinusoidal pattern. Each channel offers a different amount of frictional resistance based on the amount of surface area that contacts the catheter tube. Inertial dampers that have multiple channels with different wall geometries and widths, like inertial damper 220ʺʺ, provide versatility that can accommodate a broad range of tube sizes. Multiple channels with different wall geometries and/or widths also allow the user to test different channels and determine which channel(s) provide the best fit for securing a particular catheter tube.

CSDs according to the present disclosure are configured to be covered by bio-occlusive dressings, as noted above, which can be supplied with the CSD as a kit or packaged separately. Referring back to Figs. 9, 11 and 13, CSD 200 includes an integrated a bio-occlusive dressing 80'. Dressing 80' is attached to pad 210 via a hinge 215 section. Hinge section 215 is attached to pad 210 by a piece of double-sided tape 233. During use, bio-occlusive dressing 80' is pivotable relative to pad 210 between an open position, in which the dressing does not cover the pad and foam body 220, and a closed position, in which the dressing is folded over so that the dressing covers the entire pad and foam body. Fig. 9 shows dressing 80' in the closed position, and Fig. 13 shows the dressing in the open position.

Dressing 80' includes a foam border 82' and a transparent window 84'. Foam border 82' is formed of 1/16 in. thick piece of medical grade polyethylene foam and includes a patient-facing side 82a' and a gripping section 82b'. Window 84' is made of a medical grade polyurethane film. Foam border 82' is attached to window 84' by a third adhesive 83' applied to patient-facing side 82a' of the foam border. Window 84' has a fourth adhesive 85' applied to a patient-facing side 84a' to attach dressing 80' to foam body 220, pad 210 and catheter tube CT' as shown. Dressing 80' also has a release liner 86' applied to patient-facing side 84a' of window 84' to cover fourth adhesive 85' until the dressing is ready to be used. The total area of dressing 80' is larger than the upper surface area of pad 210, allowing window 84' to cover the entire footprint of the pad and foam body 220 with all components visible through the window as shown.

CSDs according to the present disclosure can have integrated or attached dressings, like the example in Fig. 13, or work with separate dressings. Referring again to Fig. 16, pad 210‴ and inertial damper 220‴ do not have a lateral extension for attaching a hinge portion of a dressing. Pad 210‴ and inertial damper 220‴ do not connect to an integrated dressing, but instead are parts of a standalone CSD that can be used with separately-packaged dressings.

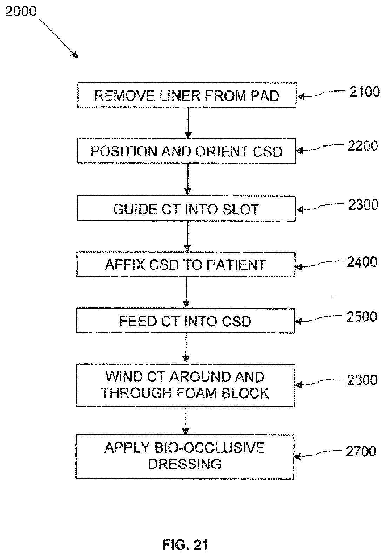

Fig. 21 illustrates another possible method 2000 for using a CSD according to the present disclosure. The following section describes steps of method 2000 assuming that CSD 200 is used, with dressing 80' in the open position. It will be appreciated that the following steps can be used with other CSDs having similar designs to CSD 200, and are not exclusively limited to CSD 200. The following section further assumes that the catheter insertion site S' is prepared according to appropriate protocol before performing the described steps.

In step 2100, liner 214 is removed from pad 210 to expose first adhesive 213 on bottom surface 211 of the pad.

In step 2200, CSD 200 is positioned over insertion site S' and rotated to the proper orientation. The user grips one side of pad 210 with a first hand (either left hand or right hand) and holds CSD 200 so that notch 218 opens or faces toward the user. This orientation is represented in Fig. 12. CSD 200 is then moved to a position in which notch 218 is in proximity to catheter tube CT'.

In step 2300, catheter tube CT' is carefully guided into notch 218 and slot 226. This step is preferably performed without allowing bottom surface 211 of pad 210 and first adhesive 213 to contact the patient. With their first hand still gripping pad 210, the user grips catheter tube CT' with their other hand and raises the catheter tube as needed so that the catheter tube does not lie flat on the patient. Holding the catheter tube CT' in the raised position, the user maneuvers CSD 200 around the catheter tube so that the catheter tube is received into notch 218 and slot 226. The user then maneuvers CSD 200 further to align first end 226a of slot 226 above insertion site S' so that the section of the catheter tube CT' exiting the insertion site is positioned in the first end.

In step 2400, the user affixes CSD 200 to the patient around insertion site S'. The user tilts pad 210 if needed so that bottom surface 211 of pad 210 is normal to an imaginary line extending vertically from insertion site S'. The user then lowers pad 210 onto the patient's skin and presses the pad down around insertion site S' so that first adhesive 213 forms a bond with the patient's skin.

In step 2500, the user feeds catheter tube CT' into CSD 200. The user grips a section of catheter tube CT' close to insertion site S' and carefully inserts the tube section into foam body 221. More specifically, the user inserts catheter tube CT' into first end 254a of third channel 254. The user can do this by gently pulling on the catheter tube CT' to apply a small amount of tension so that the catheter tube section is straight. The straight length of tube is then positioned above third channel 254 and lowered into the channel until the tube section is between the undulating sidewalls 257 of the channel. At this stage, at least the crests 251 of the sidewalls 257 frictionally engage portions of the catheter tube section inside third channel 254.

In step 2600, the user winds catheter tube CT' around and through foam body 221. Catheter tube CT' can be wound in either a clockwise direction or a counterclockwise direction around the foam body 221 and the individual inertial dampers. In addition, catheter tube CT' can be inserted one or more times through one or more of the channels. With reference to Fig. 13, for example, the user may initially wind catheter tube CT' in the clockwise direction, working the tube around second medial inertial damper 220a2, as described previously. Catheter tube CT' is then passed through second channel 253, and wound in a clockwise direction around an outer edge of second medial inertial damper 220a1, across notch 218, and around an outer edge of first medial inertial damper 220a1. Catheter tube CT' is then inserted through first channel 252. From there, catheter tube CT' extends from first channel 252 to an infusion pump or other source of fluid to be administered to the patient. A small amount of tension can be maintained on catheter tube CT' throughout the winding process, as mentioned earlier, to keep the catheter tube straight for insertion into each channel, and to keep the catheter tube closely wound around perimeter 227 of foam body 221.

Once catheter tube CT' is wound around and through foam body 221, the catheter tube is secured against movement by inertial damping in the same way that catheter CT is secured in CSD 100. Catheter tube CT' bears against and frictionally engages at least some of the sidewalls 257 of the first, second and third channels 252-254. In addition, catheter tube CT' frictionally engages at least some of the peripheral sidewalls of the inertial dampers that comprise the perimeter of foam body 221. This allows foam body 221 to convert kinetic energy from force applied to catheter tube CT' into heat energy as a result of frictional resistance between the catheter tube and foam body. Foam body 221 also absorbs some of the force applied to catheter tube CT' by plastically or elastically deforming. First and second lateral inertial dampers 220b1, 220b2 act as coil retaining members to keep CT' closely wound around the medial inertial dampers during and after the winding process.

In step 2700, the user applies the integrated bio-occlusive dressing 80'. The user can do this by holding gripping section 82b' of foam border 82' when dressing 80' is in the open position and removing release liner 86' from window 84' to expose the fourth adhesive 85'. The user then pivots or rotates dressing 80' about hinge section 215 until the dressing is inverted with window 84' centered above foam body 221. Once widow 84' is centered above foam body 221, the user presses the edges of dressing 80' onto the patient so that fourth adhesive 85' adheres to the patient, foam body 221 and catheter tube CT', as shown in Fig. 9. Dressing 80' covers the entire footprint of CSD 200, providing a protective seal around the CSD to seal the insertion site S' from moisture and reduce the risk of infection at the insertion site.

## Claims

1. A catheter securement device (100, 200) comprising:
a base (120, 210) defining a slot (126, 226); and
at least one inertial damper (130, 220) attached to the base and extending above the slot (126, 226),
the slot (126, 226) and the at least one inertial damper (130, 220) being relatively positioned so that when the slot is aligned over a catheter insertion site (S, S', S", S‴), a catheter tube (CT, CT') exiting the catheter insertion site is receivable through the slot (126, 226) and coilable around at least a portion of the at least one inertial damper.

2. The catheter securement device (100, 200) according to claim 1, wherein the slot (126, 226) and the at least one inertial damper (130, 220) are relatively positioned so that the catheter tube (CT, CT') is coilable around the at least one inertial damper (130, 220) to fully surround the at least one inertial damper (130, 220).

3. The catheter securement device (100, 200) according to claim 1 or claim 2, wherein the at least one inertial damper (130, 220) comprises a peripheral sidewall (131a, 131b, 131c, 131d, 227) configured to frictionally engage the catheter tube (CT, CT').

4. The catheter securement device (100, 200) according to claim 3, wherein the slot (126, 226) and the peripheral sidewall (131a, 131b, 131c, 131d, 227) are relatively positioned so that when the slot (126, 226) is aligned over the catheter insertion site (S, S', S", S‴), the catheter tube (CT, CT') exiting the catheter insertion site (S, S', S", S‴) is receivable through the slot (126, 226) and coilable around at least a portion of the peripheral sidewall (131a, 131b, 131c, 131d, 227).

5. The catheter securement device (100, 200) according to claim 3 or claim 4, wherein the peripheral sidewall (131a, 131b, 131c, 131d, 227) comprises a plurality of sidewall sections (131a, 131b, 131c, 131d, 227).

6. The catheter securement device (100, 200) according to claim 5, wherein the slot (126, 226) and the peripheral sidewall (131a, 131b, 131c, 131d, 227) are relatively positioned so that when the slot (126, 226) is aligned over the catheter insertion site (S, S', S", S‴), the catheter tube (CT, CT') exiting the catheter insertion site (S, S', S", S‴) is receivable through the slot (126, 226) and coilable around some or all of the plurality of sidewall sections (131a, 131b, 131c, 131d, 227).

7. The catheter securement device (100, 200) according to claims 3-6, further comprising at least two coil retaining members (144, 145, 220b1, 220b2) configured to keep the catheter tube (CT, CT') coiled around the at least one inertial damper (130, 220).

8. The catheter securement device (100, 200) according to claim 7, wherein each of the at least two coil retaining members (144, 145, 220b1, 220b2) is configured to limit migration of the catheter tube (CT, CT') away from peripheral sidewall (131a, 131b, 131c, 131d, 227) after the catheter tube (CT, CT') is coiled around the at least one inertial damper (130, 220).

9. The catheter securement device (100) according to claim 7 or claim 8, wherein the at least two coil retaining members (144, 145) comprise at least two posts (144, 145).

10. The catheter securement device (100) according to any one of the preceding claims further comprising a cover (140), wherein the at least one inertial damper (130) is contained between the base (120) and the cover (140).

11. The catheter securement device (100) according to claim 10, wherein the at least one inertial damper (130) separates the base (120) from the cover (140) by a gap (G) that surrounds the at least one inertial damper (130, 220), the gap (G) adapted to receive the catheter tube (CT) as the catheter tube is coiled around the at least one inertial damper (130).

12. The catheter securement device (100) according to claim 11, wherein the cover (140) is tiltable relative to the base (120, 210) to increase the size of the gap (G) on one side of the at least one inertial damper (130, 220).

13. The catheter securement device (200) according to claim 7 or claim 8,
wherein the at least one inertial damper (220) comprises a first lateral inertial damper (220b1), a second lateral inertial damper (220b2), and at least one medial inertial damper (220a) between the first lateral inertial damper (220b1) and the second lateral inertial damper (220b2), and
wherein the at least two coil retaining members (220b1, 220b2) comprise the first lateral inertial damper (220b1) and the second lateral inertial damper (220b2).

14. The catheter securement device (200) according to claim 13,
wherein the first lateral inertial damper (220b1) and the at least one medial inertial damper (220a) are separated by a first channel (252),
wherein the second lateral inertial damper (220b2) and the at least one medial inertial damper (220a) are separated by a second channel (253), and
wherein the first channel (252) and the second channel (253) are each configured such that the catheter tube (CT, CT') is coilable around the at least one medial inertial damper (220a) through the first channel (252) and the second channel (253).

15. The catheter securement device (200) according to claim 14,
wherein the at least one medial inertial damper (220a) comprises a first medial inertial damper (220a1) and a second medial inertial damper (220a2),
wherein the first medial inertial damper (220a1) and the second medial inertial damper (220a2) are separated by a third channel (254), and
wherein the first channel (252), the second channel (253) and the third channel (254) are each configured such that the catheter tube (CT') is coilable around the first medial inertial damper (220a1) and the second medial inertial damper (220a2) through the first channel (252), the second channel (253) and the third channel (254).

## Patentansprüche

1. Katheterbefestigungsvorrichtung (100, 200), umfassend:
eine Basis (120, 210), die einen Schlitz (126, 226) definiert, und
mindestens einen Trägheitsdämpfer (130, 220), der an der Basis angebracht ist und sich über den Schlitz (126, 226) erstreckt,
wobei der Schlitz (126, 226) und der mindestens eine Trägheitsdämpfer (130, 220) relativ positioniert sind, so dass ein aus einer Kathetereinführungsstelle (S, S', S', S‴) austretender Katheterschlauch (CT, CT') durch den Schlitz (126, 226) aufnehmbar und um mindestens einen Abschnitt des mindestens einen Trägheitsdämpfers herum aufwickelbar ist, wenn der Schlitz über einer Kathetereinführungsstelle ausgerichtet ist.

2. Katheterbefestigungsvorrichtung (100, 200) nach Anspruch 1, wobei der Schlitz (126, 226) und der mindestens eine Trägheitsdämpfer (130, 220) relativ positioniert sind, so dass der Katheterschlauch (CT, CT') um den mindestens einen Trägheitsdämpfer (130, 220) herum aufwickelbar ist, um den mindestens einen Trägheitsdämpfer (130, 220) vollständig zu umgeben.

3. Katheterbefestigungsvorrichtung (100, 200) nach Anspruch 1 oder Anspruch 2, wobei der mindestens eine Trägheitsdämpfer (130, 220) eine Umfangsseitenwand (131a, 131b, 131c, 131d, 227) umfasst, die zum Reibeingriff des Katheterschlauchs (CT, CT') ausgestaltet ist.

4. Katheterbefestigungsvorrichtung (100, 200) nach Anspruch 3, wobei der Schlitz (126, 226) und die Umfangsseitenwand (131a, 131b, 131c, 131d, 227) relativ positioniert sind, so dass der aus der Kathetereinführungsstelle (S, S', S", S‴) austretende Katheterschlauch (CT, CT') durch den Schlitz (126, 226) aufnehmbar und um mindestens einen Abschnitt der Umfangsseitenwand (131a, 131b, 131c, 131d, 227) herum aufwickelbar ist, wenn der Schlitz (126, 226) über der Kathetereinführungsstelle (S, S', S", S‴) ausgerichtet ist.

5. Katheterbefestigungsvorrichtung (100, 200) nach Anspruch 3 oder Anspruch 4, wobei die Umfangsseitenwand (131a, 131b, 131c, 131d, 227) eine Vielzahl von Seitenwandabschnitten (131a, 131b, 131c, 131d, 227) umfasst.

6. Katheterbefestigungsvorrichtung (100, 200) nach Anspruch 5, wobei der Schlitz (126, 226) und die Umfangsseitenwand (131a, 131b, 131c, 131d, 227) relativ positioniert sind, so dass der aus der Kathetereinführungsstelle (S, S', S", S‴) austretende Katheterschlauch (CT, CT') durch den Schlitz (126, 226) aufnehmbar und um einige oder alle der Vielzahl von Seitenwandabschnitten (131a, 131b, 131c, 131d, 227) herum aufwickelbar ist, wenn der Schlitz (126, 226) über der Kathetereinführungsstelle (S, S', S", S‴) ausgerichtet ist.

7. Katheterbefestigungsvorrichtung (100, 200) nach den Ansprüchen 3 - 6, ferner umfassend mindestens zwei Wickelhalteglieder (144, 145, 220b1, 220b2), die dazu ausgestaltet sind, den Katheterschlauch (CT, CT') um den mindestens einen Trägheitsdämpfer (130, 220) gewickelt zu halten.

8. Katheterbefestigungsvorrichtung (100, 200) nach Anspruch 7, wobei jedes der mindestens zwei Wickelhalteglieder (144, 145, 220b1, 220b2) dazu ausgestaltet ist, eine Migration des Katheterschlauchs (CT, CT') von der Umfangsseitenwand (131a, 131b, 131c, 131d, 227) weg zu begrenzen, nachdem der Katheterschlauch (CT, CT') um den mindestens einen Trägheitsdämpfer (130, 220) herum gewickelt ist.

9. Katheterbefestigungsvorrichtung (100) nach Anspruch 7 oder Anspruch 8, wobei die mindestens zwei Wickelhalteglieder (144, 145) mindestens zwei Pfosten (144, 145) umfassen.

10. Katheterbefestigungsvorrichtung (100) nach einem der vorhergehenden Ansprüche, ferner umfassend eine Abdeckung (140), wobei der mindestens eine Trägheitsdämpfer (130) zwischen der Basis (120) und der Abdeckung (140) gehalten ist.

11. Katheterbefestigungsvorrichtung (100) nach Anspruch 10, wobei der mindestens eine Trägheitsdämpfer (130) die Basis (120) durch einen den mindestens einen Trägheitsdämpfer (130, 220) umgebenden Spalt (G) von der Abdeckung (140) trennt, wobei der Spalt (G) dazu ausgeführt ist, den Katheterschlauch (CT) aufzunehmen, während der Katheterschlauch um den mindestens einen Trägheitsdämpfer (130) gewickelt wird.

12. Katheterbefestigungsvorrichtung (100) nach Anspruch 11, wobei die Abdeckung (140) relativ zu der Basis (120, 210) kippbar ist, um die Größe des Spalts (G) auf einer Seite des mindestens einen Trägheitsdämpfers (130, 220) zu vergrößern.

13. Katheterbefestigungsvorrichtung (200) nach Anspruch 7 oder Anspruch 8,
wobei der mindestens eine Trägheitsdämpfer (220) einen ersten seitlichen Trägheitsdämpfer (220b1), einen zweiten seitlichen Trägheitsdämpfer (220b2) und mindestens einen medialen Trägheitsdämpfer (220a) zwischen dem ersten seitlichen Trägheitsdämpfer (220b1) und dem zweiten seitlichen Trägheitsdämpfer (220b2) umfasst und
wobei die mindestens zwei Wickelhalteglieder (220b1, 220b2) den ersten seitlichen Trägheitsdämpfer (220b1) und den zweiten seitlichen Trägheitsdämpfer (220b2) umfassen.

14. Katheterbefestigungsvorrichtung (200) nach Anspruch 13,
wobei der erste seitliche Trägheitsdämpfer (220b1) und der mindestens eine mediale Trägheitsdämpfer (220a) durch einen ersten Kanal (252) getrennt sind,
wobei der zweite seitliche Trägheitsdämpfer (220b2) und der mindestens eine mediale Trägheitsdämpfer (220a) durch einen zweiten Kanal (253) getrennt sind und
wobei der erste Kanal (252) und der zweite Kanal (253) jeweils so ausgestaltet sind, dass der Katheterschlauch (CT, CT') durch den ersten Kanal (252) und den zweiten Kanal (253) um den mindestens einen medialen Trägheitsdämpfer (220a) herum aufwickelbar ist.

15. Katheterbefestigungsvorrichtung (200) nach Anspruch 14,
wobei der mindestens eine mediale Trägheitsdämpfer (220a) einen ersten medialen Trägheitsdämpfer (220a1) und einen zweiten medialen Trägheitsdämpfer (220a2) umfasst,
wobei der erste mediale Trägheitsdämpfer (220a1) und der zweite mediale Trägheitsdämpfer (220a2) durch einen dritten Kanal (254) getrennt sind und
wobei der erste Kanal (252), der zweite Kanal (253) und der dritte Kanal (254) jeweils so ausgestaltet sind, dass der Katheterschlauch (CT') durch den ersten Kanal (252), den zweiten Kanal (253) und den dritten Kanal (254) um den ersten medialen Trägheitsdämpfer (220a1) und den zweiten medialen Trägheitsdämpfer (220a2) herum aufwickelbar ist.

## Revendications

1. Dispositif de fixation de cathéter (100, 200) comprenant :
une base (120, 210) définissant une fente (126, 226) ; et
au moins un amortisseur inertiel (130, 220) fixé à la base et s'étendant au-dessus de la fente (126, 226),
la fente (126, 226) et l'au moins un amortisseur inertiel (130, 220) étant positionnés de manière relative de sorte que, lorsque la fente est alignée sur un site d'insertion de cathéter (S, S', S", S‴), un tube de cathéter (CT, CT') sortant du site d'insertion de cathéter est recevable à travers la fente (126, 226) et enroulable autour d'au moins une partie de l'au moins un amortisseur inertiel.

2. Dispositif de fixation de cathéter (100, 200) selon la revendication 1, la fente (126, 226) et l'au moins un amortisseur inertiel (130, 220) étant positionnés relativement de sorte que le tube de cathéter (CT, CT') est enroulable autour de l'au moins un amortisseur inertiel (130, 220) pour entourer complètement l'au moins un amortisseur inertiel (130, 220).

3. Dispositif de fixation de cathéter (100, 200) selon la revendication 1 ou la revendication 2, l'au moins un amortisseur inertiel (130, 220) comprenant une paroi latérale périphérique (131a, 131b, 131c, 131d, 227) configurée pour engager par friction le tube de cathéter (CT, CT').

4. Dispositif de fixation de cathéter (100, 200) selon la revendication 3, la fente (126, 226) et la paroi latérale périphérique (131a, 131b, 131c, 131d, 227) étant positionnées relativement de sorte que, lorsque la fente (126, 226) est alignée sur le site d'insertion de cathéter (S, S', S", S"'), le tube de cathéter (CT, CT') sortant du site d'insertion de cathéter (S, S', S", S‴) est recevable à travers la fente (126, 226) et enroulable autour d'au moins une partie de la paroi latérale périphérique (131a, 131b, 131c, 131d, 227).

5. Dispositif de fixation de cathéter (100, 200) selon la revendication 3 ou la revendication 4, la paroi latérale périphérique (131a, 131b, 131c, 131d, 227) comprenant une pluralité de sections de paroi latérale (131a, 131b, 131c, 131d, 227).

6. Dispositif de fixation de cathéter (100, 200) selon la revendication 5, la fente (126, 226) et la paroi latérale périphérique (131a, 131b, 131c, 131d, 227) étant positionnées relativement de sorte que, lorsque la fente (126, 226) est alignée sur le site d'insertion de cathéter (S, S', S", S‴), le tube de cathéter (CT, CT') sortant du site d'insertion de cathéter (S, S', S", S‴) est recevable à travers la fente (126, 226) et enroulable autour d'une partie ou de la totalité de la pluralité de sections de paroi latérale (131a, 131b, 131c, 131d, 227).

7. Dispositif de fixation de cathéter (100, 200) selon les revendications 3 à 6, comprenant en outre au moins deux éléments de retenue d'enroulement (144, 145, 220b1, 220b2) configurés pour maintenir le tube de cathéter (CT, CT') enroulé autour de l'au moins un amortisseur inertiel (130, 220).

8. Dispositif de fixation de cathéter (100, 200) selon la revendication 7, chacun des au moins deux éléments de retenue d'enroulement (144, 145, 220b1, 220b2) étant configuré pour limiter la migration du tube de cathéter (CT, CT') à l'écart de la paroi latérale périphérique (131a, 131b, 131c, 131d, 227) après que le tube de cathéter (CT, CT') est enroulé autour de l'au moins un amortisseur inertiel (130, 220).

9. Dispositif de fixation de cathéter (100) selon la revendication 7 ou la revendication 8, les au moins deux éléments de retenue d'enroulement (144, 145) comprenant au moins deux montants (144, 145).

10. Dispositif de fixation de cathéter (100) selon l'une quelconque des revendications précédentes, comprenant en outre un couvercle (140), l'au moins un amortisseur inertiel (130) étant contenu entre la base (120) et le couvercle (140).

11. Dispositif de fixation de cathéter (100) selon la revendication 10, l'au moins un amortisseur inertiel (130) séparant la base (120) du couvercle (140) par un espace (G) qui entoure l'au moins un amortisseur inertiel (130, 220), l'espace (G) étant adapté pour recevoir le tube de cathéter (CT) à mesure que le tube de cathéter est enroulé autour de l'au moins un amortisseur inertiel (130).

12. Dispositif de fixation de cathéter (100) selon la revendication 11, le couvercle (140) étant inclinable par rapport à la base (120, 210) pour augmenter la taille de l'espace (G) sur un côté de l'au moins un amortisseur inertiel (130, 220).

13. Dispositif de fixation de cathéter (200) selon la revendication 7 ou la revendication 8,
l'au moins un amortisseur inertiel (220) comprenant un premier amortisseur inertiel latéral (220b1), un deuxième amortisseur inertiel latéral (220b2), et au moins un amortisseur inertiel médian (220a) entre le premier amortisseur inertiel latéral (220b1) et le deuxième amortisseur inertiel latéral (220b2), et
les au moins deux éléments de retenue d'enroulement (220b1, 220b2) comprenant le premier amortisseur inertiel latéral (220b1) et le deuxième amortisseur inertiel latéral (220b2).

14. Dispositif de fixation de cathéter (200) selon la revendication 13,
le premier amortisseur inertiel latéral (220b1) et l'au moins un amortisseur inertiel médian (220a) étant séparés par un premier canal (252),
le deuxième amortisseur inertiel latéral (220b2) et l'au moins un amortisseur inertiel médian (220a) étant séparés par un deuxième canal (253), et
le premier canal (252) et le deuxième canal (253) étant chacun configurés de telle sorte que le tube de cathéter (CT, CT') est enroulable autour de l'au moins un amortisseur inertiel médian (220a) à travers le premier canal (252) et le deuxième canal (253).

15. Dispositif de fixation de cathéter (200) selon la revendication 14,
l'au moins un amortisseur inertiel médian (220a) comprenant un premier amortisseur inertiel médian (220a1) et un deuxième amortisseur inertiel médian (220a2),
le premier amortisseur inertiel médian (220a1) et le deuxième amortisseur inertiel médian (220a2) étant séparés par un troisième canal (254), et
le premier canal (252), le deuxième canal (253) et le troisième canal (254) étant chacun configurés de telle sorte que le tube de cathéter (CT') est enroulable autour du premier amortisseur inertiel médian (220a1) et du deuxième amortisseur inertiel médian (220a2) à travers le premier canal (252), le deuxième canal (253) et le troisième canal (254).
